# EUROPÄISCHE PATENTSCHRIFT

(11) **EP 2 190 355 B1**
(45) Veröffentlichungstag und Bekanntmachung des Hinweises auf die Patenterteilung: **02.03.2011**
(21) Anmeldenummer: 08785508.6
(22) Anmeldetag: 12.08.2008
(51) Int. Cl.: A61B 17/00, H01H 19/00, H01H 36/00, A61B 17/32

(54) **VORRICHTUNG ZUM ANTRIEB VON INSTRUMENTEN UND WERKZEUGEN UND DEREN VERWENDUNG**
DEVICE FOR DRIVING INSTRUMENTS AND TOOLS AND THE USE THEREOF
DISPOSITIF POUR L'ENTRAÎNEMENT D'INSTRUMENTS ET D'OUTILS, ET SON UTILISATION

(30) Priorität: 14.08.2007 DE 102007038358
(43) Veröffentlichungstag der Anmeldung: 02.06.2010
(73) Patentinhaber: WISAP Gesellschaft für wissenschaftlichen Apparatebau mbH, Rudolf-Diesel-Ring 20 82054 Sauerlach (DE)
(72) Erfinder: REPPENTHIEN, Joachim, 82054 Sauerlach (DE)
(74) Vertreter: Grape, Knut
(86) Internationale Anmeldenummer: PCT/EP2008/006632
(87) Internationale Veröffentlichungsnummer: WO 2009/021718

(56) Entgegenhaltungen:
- EP-A- 0 278 649
- EP-A- 1 066 930
- WO-A-80/01533
- WO-A-2007/064885
- WO-A-2007/089603
- DE-A1- 2 839 175
- DE-A1-102004 051 913
- DE-B- 1 227 544
- FR-A- 2 683 448
- US-A- 4 497 988
- US-A- 4 595 850
- US-A- 5 138 243
- US-A- 5 311 949
- US-A- 5 769 211
- US-A1- 2002 069 730
- US-A1- 2003 205 492
- US-A1- 2006 028 089
- US-B1- 6 199 642
- US-B1- 6 666 875
- US-B1- 7 034 240

## Beschreibung

Die Erfindung betrifft eine Vorrichtung zum Antrieb von chirurgischen Instrumenten.

Derartige Vorrichtungen sind allgemein bekannt. Demnach dienen solche Vorrichtungen beispielsweise auf dem medizinischen Gebiet der Gynäkologie dem Antrieb von in Drehung zu versetzenden Schneideeinrichtungen zum Entfernen von Gewebe aus einer Gebärmutter, einer Gebärmutter insgesamt oder zur Behandlung von Myomen, etc. Derartige Vorrichtungen finden darüber hinaus in der Medizin auf zahlreichen und zum Teil unterschiedlichsten weiteren Fachgebieten Verwendung, etwa bei operativen Eingriffen im Bauchraum, am Magen und zur Gallenblasenentfernung oder Blinddarmentfernung. Derartige Vorrichtungen weisen in aller Regel ein Gehäuse zur Aufnahme eines Motors, einer dem Motor zugeordneten Stromversorgungseinrichtung und einem dem Motor zugeordneten, teilweise aus dem Gehäuse herausragenden und mit den chirurgischen Instrumenten zusammenwirkenden, zapfenförmigen Antriebselement und eine an der Außenseite des Gehäuses angeordnete Schalteinrichtung zum Einschalten und Ausschalten des Motors auf. Allerdings haben sich derartige Vorrichtungen in der Praxis als verhältnismäßig nachteilig erwiesen. So besitzen derartige Vorrichtungen allesamt jeweils eine Schalteinrichtung, die ein Betätigungselement umfasst, welches ausnahmslos als Druckknopfschalter, welcher radial in das Gehäuse hinein- und herausbewegbar ist, oder als Schiebeschalter, welcher in Längsrichtung des Gehäuses hin- und herverschiebbar ist, ausgebildet ist. Ein solches Betätigungselement befindet sich mithin an einer ganz bestimmten Stelle des Gehäuses. Zu dessen Betätigung muss die Vorrichtung daher im Allgemeinen zunächst gegenüber dem Daumen oder einen Finger der Hand des Benutzers exakt ausgerichtet werden. Erst anschließend kann das Betätigungselement radial gedrückt oder in Längsrichtung verschoben werden. Eine derart notwendige Handhabung ist zum einen aufwendig und verhindert zum anderen einen zeitnahen Eingriff, was insbesondere bei Operationen auf medizinischem Gebiet hinderlich ist.

Die FR-PS 2 683 448 beschreibt eine Vorrichtung zum Antrieb einer Zahnbürste, die ein Gehäuse zur Aufnahme eines Motors, einer dem Motor zugeordneten Stromversorgungseinrichtung und einem dem Motor zugeordneten, teilweise aus dem Gehäuse herausragenden und mit der Zahnbürste zusammenwirkenden, zapfenförmigen Antriebselement und eine an der Außenseite des Gehäuses angeordnete Schalteinrichtung zum Einschalten und Ausschalten des Motors aufweist, wobei die Schalteinrichtung ein Betätigungselement, das sich vollständig um den Umfang des Gehäuses erstreckt und gegenüber dem Gehäuse relativ verdrehbar ausgebildet ist, umfasst. Diese bekannte Vorrichtung unterscheidet sich grundlegend. In konstruktiver Hinsicht umfasst die Schalteinrichtung zwar ein Betätigungselement, das sich vollständig um den Umfang des Gehäuses erstreckt und gegenüber dem Gehäuse relativ verdrehbar ausgebildet ist, ohne dass dafür allerdings eine technische Notwendigkeit erkennbar ist. So scheint die Ausbildung des Betätigungselementes eher zufällig gewählt und von rein dekorativer Natur zu sein. Auch weist diese Vorrichtung weder einen Magnetschalter zum Einschalten und Ausschalten des Motors noch ein dem Motor zugeordnete und als Motorbremse für den Motor vorgesehenes Relais auf.

Die US-A-2002/0069730, zeigt eine Drehmomenten-Werkzeuganordnung mit einem Hebel zur Aktivierung bzw. Deaktivierung eines Hall-Sensors zum Ein- und Ausschalten eines Elektromotors.

Die DE 1 227 544 B beschreibt schließlich ganz allgemein eine Betätigungseinrichtung für einen durch Magnetkraft zu betätigenden Kontakt.

Die WO-A-8001533, US-A-4595850 und US-A-2003/0205492 zeigen Zahnbürsten bzw. Teile davon mit unter anderem einem Magnetschalter als Schalteinrichtung. Ein Relais, das dem Motor zugeordnet und als Motorbremse sowie zum Aufbau und zur Aufrechterhaltung einer ausreichenden Betriebsspannung dient, ist weder vorgesehen noch konstruktiv überhaupt erforderlich.

Die US-A-5769211 offenbart eine Vorrichtung zum Antrieb von chirurgischen Instrumenten. Die Schalteinrichtung ist ohne einen durch das Betätigungselement betätigbaren Magnetschalter zum Einschalten und Ausschalten des Motors und ein dem Motor zugeordnetes und als Motorbremse sowie zum Aufbau und zur Aufrechterhaltung einer ausreichenden Betriebsspannung vorgesehenes Relais ausgebildet.

Der Erfindung liegt daher die Aufgabe zugrunde, eine Vorrichtung zum Antrieb von chirurgischen Instrumenten zur Verfügung zu stellen, mit welcher sich die obigen Nachteile verhindern lassen, welche mithin konstruktiv besonders einfach, kompakt und stabil ausgebildet ist und gleichzeitig eine ausgesprochen einfache und schnelle Handhabung ermöglicht.

Diese Aufgabe wird auf überraschend einfache Weise durch die Merkmale des Anspruchs 1 gelöst.

Demnach lässt sich durch die Ausgestaltung der erfindungsgemäßen Vorrichtung zum Antrieb von chirurgischen Instrumenten, die ein Gehäuse zur Aufnahme eines Motors, den Motor, eine dem Motor zugeordnete Stromversorgungseinrichtung und ein dem Motor zugeordnetes, teilweise aus dem Gehäuse herausragendes und mit den Instrumenten zusammenwirkendes, zapfenförmiges Antriebselement sowie eine an der Außenseite des Gehäuses angeordnete Schalteinrichtung zum Einschalten und Ausschalten des Motors aufweist, wobei die Schalteinrichtung ein Betätigungselement, das sich vollständig um den Umfang des Gehäuses erstreckt und gegenüber dem Gehäuse relativ verdrehbar ausgebildet ist, und wobei die Schalteinrichtung einen durch das Betätigungselement betätigbaren Magnetschalter zum Einschalten und Ausschalten des Motors und ein dem Motor zugeordnetes und als Motorbremse sowie zum Aufbau und zur Aufrechterhaltung einer ausreichenden Betriebsspannung vorgesehenes Relais umfasst, eine einfache, kompakte und stabile Bauweise der gesamten Vorrichtung erreichen. Durch die Ausgestaltung der erfindungsgemäße Vorrichtung mit einem sich vollständig um den Umfang des Gehäuses erstreckenden und gegenüber dem Gehäuse relativ verdrehbar ausgebildeten Betätigungselement der Schalteinrichtung ergibt sich zudem eine ausgesprochen einfache und schnelle Handhabung der Vorrichtung insgesamt. So muss die Vorrichtung bei deren Gebrauch nicht erst in Stellung gebracht werden, um das Betätigungselement gegenüber dem Daumen oder einen Finger der Hand des Benutzers auszurichten. Dadurch, dass sich das Betätigungselement wenigstens teilweise um den Umfang des Gehäuses erstreckt und gegenüber dem Gehäuse relativ verdrehbar ist, ist ein gesondertes, aufwendiges Instellungbringen der Vorrichtung nicht erforderlich. Allenfalls ist mit einem Handgriff eine lediglich geringe Verdrehung der Vorrichtung vorzunehmen, die um so geringer ausfällt, um so mehr sich das Betätigungselement um den Umfang des Gehäuses erstreckt. Damit einhergehend ist die Handhabung der Vorrichtung nach der Erfindung für Benutzer, die Rechtshänder sind, gleichermaßen wie für Benutzer, die Linkshänder sind, erheblich vereinfacht. Durch den Magnetschalter ist erfindungsgemäß zusätzlich eine Leichtgängigkeit der Schalteinrichtung sichergestellt, da die einzelnen Bauteile in keinem mechanischen Eingriff zueinander stehen. Zugleich ist mechanischer Abrieb und eine damit einhergehende Reparaturanfälligkeit der einzelnen Bauteile zueinander vermindert. Beides erhöht die Lebensdauer der Schalteinrichtung, was sich wiederum sehr vorteilhaft auf die erfindungsgemäße Vorrichtung insgesamt auswirkt, vor allem dann, wenn die Vorrichtung ein gekapseltes Gehäuse aufweist. Schließlich hat der Magnetschalter den besonderen Vorteil, gegenüber Flüssigkeit, Dampf und/oder Gas, welche zur Sterilisation der Vorrichtung nach erfolgter Benutzung Verwendung finden, unempfindlich und absolut dicht zu sein. Schließlich lassen sich durch das dem Motor zugeordnete Relais darüber hinaus erfindungsgemäß zwei besonders wichtige Funktionen erreichen:

Zum einen ist das Relais als Motorbremse für den Motor vorgesehen. Zum anderen dient das Relais dazu, eine ausreichende Betriebsspannung zum Motor bzw. Kontaktdraht der Schalteinrichtung aufzubauen und aufrechtzuerhalten. Insoweit erfolgt ein Einschalten bzw. Ausschalten des Motors nahezu in Echtzeit. Ein sogenanntes Nachlaufen des Motors ist ausgeschlossen.

Weitere vorteilhafte Einzelheiten der erfindungsgemäßen Vorrichtung sind in den Ansprüchen 2 bis 20 beschrieben.

Von ganz besonderer Bedeutung sind die Merkmale des Anspruchs 2, wonach das Betätigungselement der Schalteinrichtung ring-oder scheibenförmig ausgebildet ist. Das Betätigungselement der Schalteinrichtung erstreckt sich somit um den gesamten Umfang des Gehäuses der erfindungsgemäßen Vorrichtung. Das Betätigungselement der Schalteinrichtung ist mithin unabhängig von der Vorrichtung selbst jederzeit ungehindert zugänglich. Eine Instellungbringung oder Ausrichtung der Vorrichtung in der Hand des Benutzers entfällt gänzlich. Ebenso vereinfacht sich die Handhabung für einen Benutzer, der Linkshänder wie ebenso Rechtshänder ist, wesentlich. In besonders vorteilhafter Weise ist das Betätigungselement der Schalteinrichtung als Ring ausgebildet ist, der insbesondere an einem Teil des Gehäuses abgestützt ist. Hierdurch erhält das Betätigungselement eine zusätzliche Stabilität, welche der gesamten Vorrichtung der Erfindung bei deren Handhabung zugute kommt.

Das Betätigungselement der Schalteinrichtung erstreckt sich nach den Maßnahmen des Anspruchs 3 über die Außenseite des Gehäuses hinaus, wodurch die Handhabbarkeit der erfindungsgemäßen Vorrichtung durch eine ausgesprochen einfache Erreichbarkeit des Betätigungselementes der Schalteinrichtung selbst ohne Inbetrachtungsnahme, d.h. durch reines Abtasten, verbessert wird.

Weiterhin liegt es im Rahmen der Erfindung, dass das Betätigungselement der Schalteinrichtung nach Anspruch 4 an dessen Außenumfang mit einer Profilierung versehen ist. Aufgrund einer solchen Profilierung lässt sich die Handhabung der Vorrichtung nach der Erfindung durch Erhöhung der Griffigkeit des Betätigungselementes noch zusätzlich verbessern. Ein versehentliches Abrutschen des Daumes oder jeweiligen Fingers des Benutzers von dem Betätigungselement ist daher sicher ausgeschlossen. Zu diesem Zweck ist die Profilierung in vorteilhafter Weise an dem Außenumfang des Betätigungselementes der Schalteinrichtung als Rändelung, Zahnrad oder Zahnkranz ausgebildet. Alternativ dazu kann die Profilierung auch in Form von wenigstens zwei Griffmulden oder dergleichen ausgestaltet sein, wobei dann bevorzugt jedoch mindestens vier Griffmulden, also jeweils eine Griffmulde in einer Teilung von 90°, vorgesehen sein sollte.

In weiterer Ausgestaltung der Erfindung ist das Betätigungselement der Schalteinrichtung nach Anspruch 5 gegenüber dem Gehäuse um die Längsachse des Gehäuses oder eine dazu parallele Achse relativ verdrehbar ausgebildet.

Des Weiteren ist erfindungsgemäß vorgesehen, dass das Betätigungselement der Schalteinrichtung nach Anspruch 6 aus einer ersten Stellung, insbesondere Ausgangsstellung, in eine zweite Stellung, insbesondere Kontaktstellung, und umgekehrt, gegenüber dem Gehäuse relativ verdrehbar ist.

Zur konstruktiven Vereinfachung der erfindungsgemäßen Vorrichtung einerseits und zur gleichzeitigen Verbesserung von Kompaktheit und Stabilität der erfindungsgemäßen Vorrichtung andererseits dienen die Merkmale der Ansprüche 7 bis 9.

Demzufolge ist dem Betätigungselement der Schalteinrichtung nach Anspruch 7 wenigstens ein Anschlagelement zur Begrenzung der relativen Verdrehbarkeit des Betätigungselementes gegenüber dem Gehäuse zugeordnet. Mit einer solchen Ausbildung ist auf einfache Weise erreicht, dass das Betätigungselement der Schalteinrichtung zwischen den zwei Stellungen, nämlich der Ausgangsstellung und der Kontaktstellung, hin- und herverdrehbar ist. Die Drehbewegung des Betätigungselementes der Schalteinrichtung ist daher exakt definiert.

In diesem Zusammenhang ist erfindungsgemäß vorgesehen, das Betätigungselement der Schalteinrichtung entsprechend den Merkmalen des Anspruchs 8 mit wenigstens einem/einer im Wesentlichen bogenförmigen Langloch oder Ausnehmung zu versehen, in dem/der ein sich im Wesentlichen parallel zu der Längsachse des Gehäuses verlaufender Stift, Bolzen oder dergleichen Führungselement aufgenommen ist.

Bevorzugt ist das Betätigungselement der Schalteinrichtung nach Anspruch 9 mit zwei im Wesentlichen bogenförmigen Langlöchern oder Ausnehmungen versehen, in denen jeweils ein sich im Wesentlichen parallel zu der Längsachse des Gehäuses verlaufender Stift, Bolzen oder dergleichen Führungselement aufgenommen ist. Dass die zwei bogenförmigen Langlöcher oder Ausnehmungen an dem Betätigungselement der Schalteinrichtung im Wesentlichen um etwa 180° zueinander versetzt angeordnet sind, dient zusätzlich einer exakten Führung und Begrenzung des Betätigungselementes bei dessen Verdrehung relativ zu dem Gehäuse.

In vorteilhafter Weise umfasst die Schalteinrichtung entsprechend den konstruktiven Maßnahmen des Anspruchs 10 einen Reed-Schalter zum Einschalten und Ausschalten des Motors. Dabei umfasst die Schalteinrichtung nach Anspruch 11 vorzugsweise einen Kontaktdraht zur elektrischen Verbindung des Motors mit der Stromversorgungseinrichtung und einen mit dem Kontaktdraht zusammenwirkenden Magnet, wobei insbesondere der Kontaktdraht der Schalteinrichtung im Wesentlichen in einer sich etwa parallel zur Längsachse des Gehäuses erstreckenden Nut und der Magnet an dem Betätigungselement der Schalteinrichtung an dessen Innenumfang dem Kontaktdraht zugewandt angeordnet sind, und umgekehrt.

Zur weiteren Vereinfachung der Handhabung der erfindungsgemäßen Vorrichtung umfasst die Schalteinrichtung nach Anspruch 12 eine Rückstelleinrichtung, welche dem Betätigungselement zugeordnet ist und das Betätigungselement beaufschlagt, derart, dass das Betätigungselement selbsttätig aus der Kontaktstellung in die Ausgangsstellung zurück verbringbar ist.

In diesem Zusammenhang ist es besonders vorteilhaft, dass die Rückstelleinrichtung nach Anspruch 13 eine Magnetanordnung aufweist, wobei ein Magnet an oder in dem Betätigungselement der Schalteinrichtung angeordnet ist und zwei dem einen Magnet zugewandte Magnete in dem Gehäuse angeordnet sind. In alternativer Ausgestaltung kann die Rückstelleinrichtung eine Federanordnung aufweisen, wobei eine Feder zwischen dem Betätigungselement der Schalteinrichtung und dem Gehäuse angeordnet ist.

Von ausgesprochen großer Bedeutung sind weiterhin die konstruktiven Maßnahmen des Anspruchs 14. Danach umfasst die Schalteinrichtung eine Einrichtung zum Anzeigen des aktuellen Zustandes der dem Motor zugeordneten Stromversorgungseinrichtung. Ebenso denkbar ist es, mittels einer solchen Einrichtung die verbleibende und damit dem Benutzer noch zur Verfügung stehende Ladung eines Akkumulators bzw. einer Batterie oder auch den momentanen Aktivitätszustand beim Laden eines Akkumulators anzuzeigen.

Dabei ist erfindungsgemäß vorgesehen, dass die Schalteinrichtung nach Anspruch 15 wenigstens einen lichtdurchlässigen Gehäuseabschnitt, der mit optischen Signalgebungselementen, insbesondere lichtemittierenden Dioden, versehen ist, umfasst. Insbesondere ist dabei der wenigstens eine lichtdurchlässige Gehäuseabschnitt kontinuierlich, insbesondere ringförmig, ausgebildet. In alternativer oder kumulativer Ausgestaltung kann der wenigstens eine lichtdurchlässige Gehäuseabschnitt ebenso diskontinuierlich, insbesondere punktförmig, ausgebildet sein.

Nach Anspruch 16 ist das Gehäuse in ganz bevorzugter Ausgestaltung gekapselt ausgebildet, wobei die Stromversorgungseinrichtung wenigstens einen wiederaufladbaren Akkumulator aufweist. Durch eine solche gekapselte Ausführung des Gehäuses ist eine hohe Lebensdauer der erfindungsgemäßen Vorrichtung erreicht, da sich die Vorrichtung ohne jede Funktionsbeeinträchtigung vielfach mittels Flüssigkeit, Dampf und/oder Gas sterilisieren und somit für nachfolgende Einsätze und Operationen wiederverwenden lässt. Einem Eintritt von Feuchtigkeit in das Innere der Vorrichtung nach der Erfindung ist durch eine Kapselung des Gehäuses sicher entgegengewirkt.

In diesem Zusammenhang ist erfindungsgemäß vorgesehen, dass der wenigstens eine wiederaufladbare Akkumulator nach Anspruch 17 über an einer Außenseite des Gehäuses angeordnete Kontakte, insbesondere einen kreisringförmigen Kontakt, an einem externen Ladegerät elektrisch anschließbar ist.

In alternativer Ausgestaltung dazu kann das Gehäuse nach Anspruch 18 gekapselt ausgebildet sein, wobei die Stromversorgungseinrichtung eine Leitung zur elektrischen Verbindung mit einer externen Stromquelle aufweist, oder über einen Schraub-, Bayonette- oder dergleichen -verschluss mit einem deckel- oder kappenförmigen Verschlusselement abgedichtet verschließbar sein, wobei die Stromversorgungseinrichtung wenigstens eine Batterie aufweist.

In besonders vorteilhafter Weise kann/können das Gehäuse und/oder die Schalteinrichtung und/oder das Betätigungselement und/oder der lichtdurchlässige Gehäuseabschnitt nach Anspruch 19 aus Kunststoff, insbesondere (Hart-)Polyvinylchlorid, Polyoximethylen (POM), Polyester, Polyphenylensulfon (PPSU), ABS, Acryl, Polycarbonat, Tetrafluorethylen oder Impax, duroplastischen Elastomeren, mit oder ohne Glasfaserverstärkung, der einer Kombination hieraus gebildet sein. Die erfindungsgemäße Vorrichtung ist besonders leichtbauend und lässt sich ausgesprochen kostengünstig herstellen. Alternativ dazu liegt es ebenso im Rahmen der Erfindung, dass das Gehäuse und/oder die Schalteinrichtung und/oder das Betätigungselement nach Anspruch 19 aus Metall, insbesondere (nicht-rostendem) Stahl, Edelstahl, Aluminium, Messing, Zink, Rotgusslegierungen oder einer Legierung hieraus gebildet ist/sind. Die Vorrichtung lässt sich somit insgesamt thermisch und/oder chemisch sterilisieren und ohne weiteres für den Mehrfach-Gebrauch wiederverwenden.

Schließlich dienen die Merkmale des Anspruchs 20, wonach das Gehäuse mittels Fräsens, Spritzgießens oder Laser herstellbar ist, einer vielseitigen wie gleichermaßen einer an individuelle Gegebenheiten speziell angepassten Verwendbarkeit sowie kostengünstigen Herstellung der erfindungsgemäßen Vorrichtung.

Die Vorrichtung zum Antrieb von chirurgischen Instrumenten kann zu laparaskopischen, thorakoskopischen oder athroskopischen und minimal-invasiven chirurgischen Eingriffen verwendet werden. So eignet sich die Vorrichtung beispielsweise zum Antrieb von Morcellatoren, um Gewebezylinder aus einem Gewebe, das sich innerhalb einer Körper- oder Gelenkhöhle und/oder in bzw. an einem Wandbereich davon befindet, beispielsweise eines von einem organischen Hohlraum eines menschlichen Körpers wenigstens teilweise aufgenommenen und/oder umgebenen, organischen Körpers, vorzugsweise von Embryonen, Myomen, Geschwülsten, Geschwüren und Karzinomen auszuschneiden und zu entfernen.

Weitere Merkmale, Vorteile und Einzelheiten der Erfindung ergeben sich aus der nachfolgenden Beschreibung einiger bevorzugter Ausführungsformen der Erfindung sowie anhand der Zeichnungen. Hierbei zeigen:
- Fig. 1.: eine perspektivische Vorderansicht einer Ausführungs- form einer erfindungsgemäßen Vorrichtung zum Antrieb von chirurgischen Instrumenten,
- Fig. 2: eine perspektivische Rückansicht der Ausführungsform der erfindungsgemäßen Vorrichtung zum Antrieb von chirurgischen Instrumenten nach der Fig. 1,
- Fig. 3 und 4: eine Draufsicht und eine Unteransicht auf die Ausführungsform der erfindungsgemäß ausgebildeten Vorrichtung nach der Fig. 1,
- Fig. 5: eine Seitenansicht der Ausführungsform der erfin- dungsgemäß ausgebildeten Vorrichtung entsprechend den Fig. 1 bis 4,
- Fig. 6: eine Querschnittansicht durch die Ausführungsform der erfindungsgemäß ausgebildeten Vorrichtung längs der Linie VI-VI in der Fig. 5,
- Fig. 7: eine Querschnittansicht durch die Ausführungsform der erfindungsgemäß ausgebildeten Vorrichtung längs der Linie VII-VII in der Fig. 5,
- Fig. 8A bis 8E: eine aufgebrochene schematische, perspekti- vische Vorderansicht, Draufsicht, perspektivische Rückansicht und Unteransicht sowie Seitenansicht der Ausführungsform der erfindungsgemäß ausgebildeten Vorrichtung entsprechend den Fig. 1 bis 7,
- Fig. 9A bis 9F: eine perspektivische Ansicht und eine Sei- tenansicht einer anderen Ausführungsform einer erfin- dungsgemäß ausgebildeten Vorrichtung und einer Drauf- sicht, einer weiteren Seitenansicht sowie zwei Quer- schnittansichten durch die Ausführungsform der Vor- richtung längs der Linien IXE-IXE und IXF-IXF in der Fig. 9D,
- Fig. 10 bis 16: eine perspektivische Vorderansicht, eine perspektivische Rückansicht, eine Draufsicht, eine Unteransicht und eine Seitenansicht einer noch ande- ren Ausführungsform einer erfindungsgemäßen Vorrich- tung zum Antrieb von chirurgischen Instrumenten sowie eine Querschnittansicht durch die Ausführungsform der erfindungsgemäß ausgebildeten Vorrichtung längs der Linie XV-XV in der Fig. 14 und eine Querschnittan- sicht durch die Ausführungsform der erfindungsgemäß ausgebildeten Vorrichtung längs der Linie XVI-XVI in der Fig. 14, entsprechend den Fig. 1 bis 7, und
- Fig. 17 bis 21: Querschnittansichten durch die Ausführungs- form der erfindungsgemäß ausgebildeten Vorrichtung nach den Fig. 10 bis 16 längs den Linien XVII-XVII, XVIII-XVIII und XIX-XIX in der Fig. 14 und längs den Linien XX-XX und XXI-XXI in der Fig. 12.

Die erfindungsgemäße Vorrichtung 10 ist zum Antrieb von chirurgischen Instrumenten vorgesehen. Bei der nachfolgenden Beschreibung einiger Ausführungsbeispiele der erfindungsgemäßen Vorrichtung 10 sind einander entsprechende, gleiche Bauteile jeweils mit identischen Bezugsziffern versehen.

Die - insbesondere tragbare - Vorrichtung 10 nach der Erfindung eignet sich zum Antrieb von chirurgischen Instrumenten im Allgemeinen. Darüber hinaus eignet sich die Vorrichtung 10 nach der Erfindung zum Antrieb von medizinischen Instrumenten im Besonderen, wie etwa zum Antrieb von in Drehung zu versetzenden Schneideeinrichtungen zum Operieren, insbesondere Entfernen, eines organischen Gewebes, vorzugsweise von Myomen, Geschwülsten, Geschwüren, Karzinomen etc., oder eines anorganischen Körpers, wie Gallen- und Blasensteinen oder dergleichen Agglomerationen, die von einer Körper- oder Gelenkhöhle bzw. einem pers und/oder in bzw. an einem Wandbereich davon wenigstens teilweise aufgenommen und/oder umgeben sind.

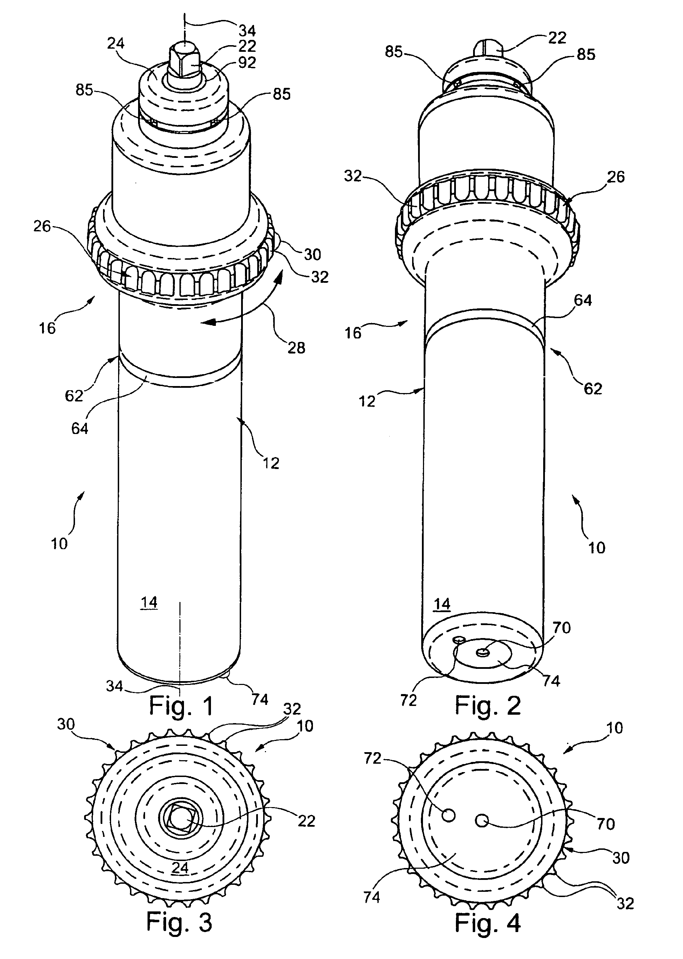

In den Fig. 1 bis 8E ist eine bevorzugte Ausführungsform einer solchen Vorrichtung 10 nach der Erfindung dargestellt.

Die Vorrichtung 10 weist ein Gehäuse 12 und eine an der Außenseite 14 des Gehäuses 12 angeordnete Schalteinrichtung 16 auf.

Wie insbesondere den Fig. 1 bis 8E zu entnehmen ist, ist das Gehäuse 12 im Wesentlichen zylinderförmig ausgebildet. Ohne im Einzelnen dargestellt zu sein, kann das Gehäuse 12 alternativ oder kumulativ eine an eine Hand eines Benutzers angepasste, anatomische Form ausweisen.

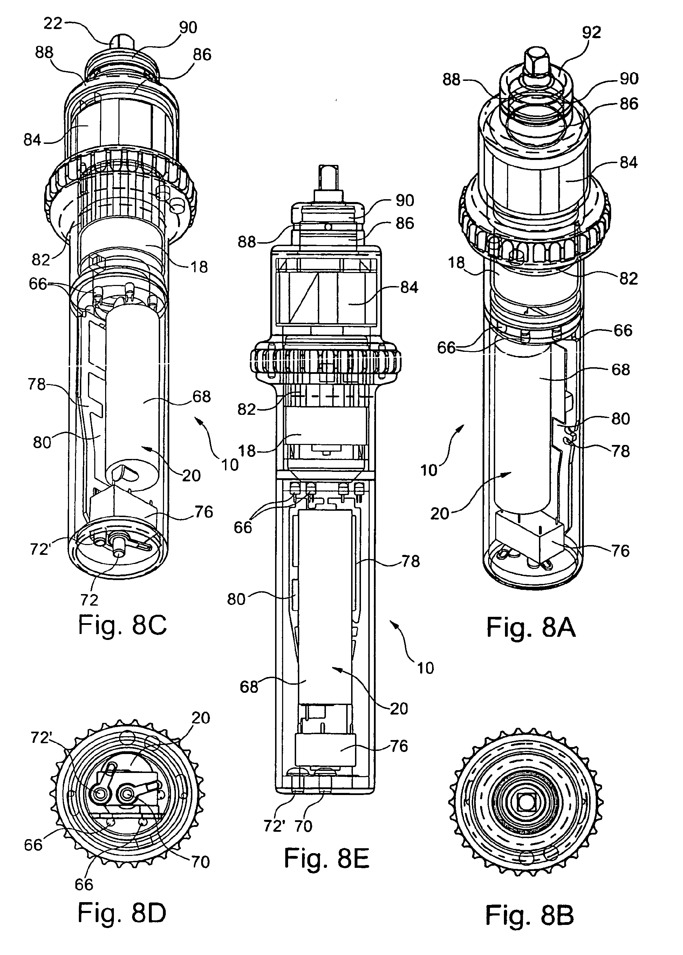

Das Gehäuse 12 nimmt entsprechend den Fig. 8A bis 8E einen Motor 18, eine Stromversorgungseinrichtung 20, die dem Motor 18 zugeordnet ist, und ein zapfenförmiges Antriebselement 22 auf. Das zapfenförmige Antriebselement 22 ist ebenfalls dem Motor 18 zugeordnet, ragt teilweise aus dem Gehäuse 12, nämlich aus der oberen Stirnseite 24 der Außenseite 14 des Gehäuses 12, heraus und wirkt mit den Instrumenten oder Werkzeugen (nicht dargestellt), welche angetrieben werden sollen, zusammen.

Die Schalteinrichtung 16 dient dem Einschalten und Ausschalten des Motors 18. Dabei umfasst die Schalteinrichtung 16 ein Betätigungselement 26.

Das Betätigungselement 26 erstreckt sich wenigstens teilweise, und insbesondere vollständig, um den Umfang des zylinderförmig ausgeformten Gehäuses 12 und ist gegenüber dem Gehäuse 12 entsprechend Doppelpfeil 28 relativ verdrehbar ausgebildet.

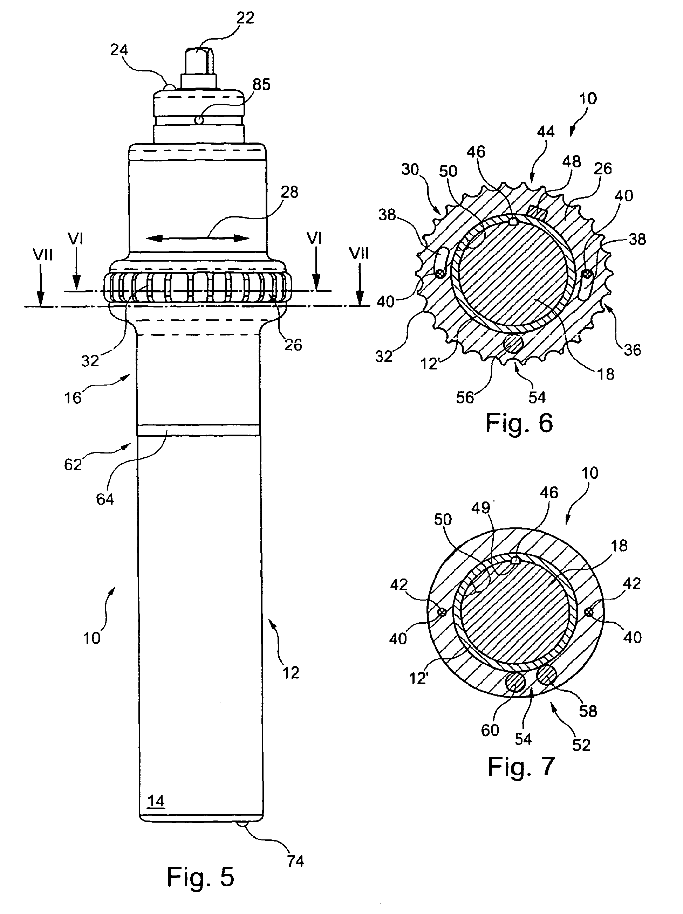

Bei der Ausführungsform der erfindungsgemäßen Vorrichtung 10, die in den Fig. 1 bis 8E gezeigt ist, ist das Betätigungselement 26 der Schalteinrichtung 16 ring- oder scheibenförmig ausgebildet. Vorzugsweise ist das Betätigungselement 26 der Schalteinrichtung 16 entsprechend der Fig. 6 als Ring ausgebildet, der in vorteilhafter Weise an einem Teil 12' des Gehäuses 12 abgestützt ist. Wie den Fig. 6 und 7 entnehmbar ist, ist der Teil 12' des Gehäuses 12 ebenfalls etwa zylinderförmig ausgestaltet.

Bei der in den Fig. 1 bis 8E dargestellten Ausführungsform der Vorrichtung 10 ragt das Betätigungselement 26 mit dessen Außenumfang 30 über die Außenseite 14 des Gehäuses 12 hinaus. Das Betätigungselement 26 weist insoweit einen größeren Außendurchmesser als das Gehäuse 12 selbst auf. Auf diese Weise lässt sich das Betätigungselement 26 einfach, ohne großen Aufwand und schnell ergreifen und zur weiteren Handhabung der Vorrichtung 10 betätigen.

Des Weiteren ist das Betätigungselement 26 der Schalteinrichtung 16 an dessen Außenumfang 30 mit einer Profilierung 32 versehen. Die Profilierung 32 an dem Außenumfang 30 ist als Rändelung, Zahnrad, Zahnkranz oder dergleichen ausgebildet.

Das Betätigungselement 26 der Schalteinrichtung 16, ist in vorteilhafter Weise gegenüber dem Gehäuse 12 um die Längsachse 34 des Gehäuses oder eine dazu parallele Achse relativ verdrehbar ausgebildet.

Weiterhin ist das Betätigungselement 26 der Schalteinrichtung 16 aus einer ersten Stellung, insbesondere Ausgangsstellung, welche in der Fig. 6 schematisch dargestellt ist, in eine zweite Stellung, insbesondere Kontaktstellung (nicht dargestellt), und umgekehrt, gegenüber dem Gehäuse 12 relativ verdrehbar.

Zur Begrenzung der relativen Verdrehbarkeit des Betätigungselementes 26 gegenüber dem Gehäuse 12 ist dem Betätigungselement 26 entsprechend den Fig. 6 und 7 wenigstens ein Anschlagelement 36 zugeordnet. Das Anschlagelement 36 umfasst bei der Ausführungsform der Vorrichtung 10, die in den Fig. 6 und 7 gezeigt ist, wenigstens ein im Wesentlichen bogenförmiges Langloch 38 bzw. eine entsprechend ausgeformte Ausnehmung, in dem/der ein Stift 40, Bolzen oder dergleichen Führungs- bzw. Kulissenelement aufgenommen ist. Der Stift 40, Bolzen oder dergleichen Führungselement verläuft parallel zu der Längsachse 34 des Gehäuses 12.

Bei dem Ausführungsbeispiel der Vorrichtung 10 nach den Fig. 6 und 7 ist das wenigstens eine Anschlagelement 36 in Form von zwei im Wesentlichen bogenförmigen Langlöchern 38 oder Ausnehmungen ausgestaltet, in denen jeweils ein sich parallel zu der Längsachse 34 des Gehäuses 12 verlaufender Stift 40, Bolzen oder dergleichen Führungselement aufgenommen und geführt ist. Der Stift 40, Bolzen oder dergleichen Führungselement ist jeweils endseitig in einer Bohrung 42 passgenau eingebracht und aufgenommen.

Die Länge des im Wesentlichen bogenförmigen Langloches 38 oder dergleichen Ausnehmung bestimmt die Lage der ersten Stellung bzw. Ausgangsstellung und der zweiten Stellung bzw. Kontaktstellung des Betätigungselementes 26.

Vorzugsweise sind die zwei bogenförmigen Langlöcher 38 oder Ausnehmungen an dem Betätigungselement 26 im Wesentlichen um 180° zueinander versetzt angeordnet, also über den Umfang des als Ring ausgebildeten Betätigungselementes 26 gleichmäßig verteilt angeordnet. Auf diese Weise lässt sich eine exakte Führung und zugleich Begrenzung der Drehbewegung des Betätigungselementes 26 erreichen.

Des Weiteren umfasst die Schalteinrichtung 16 einen Magnetschalter 44, der vorzugsweise als Reed-Schalter ausgebildet ist, um den Motor 18 einzuschalten und/oder auszuschalten, und umgekehrt.

Insbesondere umfasst die Schalteinrichtung einen Kontaktdraht 46 zur elektrischen Verbindung des Motors 18 mittels Stromversorgungseinrichtung 20 und einen mit dem Kontaktdraht 46 zusammenwirkenden Magnet 48. Der Kontaktdraht 46 der Schalteinrichtung 16 ist in einer Nut 49 angeordnet, die sich etwa parallel zur Längsachse 34 des Gehäuses 12 erstreckt. Der Magnet 48 ist demgegenüber an dem Betätigungselement 26 der Schalteinrichtung 16 angeordnet, und zwar an dem Innenumfang 50 und dem Kontaktdraht 46 zugewandt. Ohne im Einzelnen dargestellt zu sein, ist durchaus auch eine kinematische Umkehr dessen denkbar, gleichwohl eine solche konstruktive Ausgestaltung einen erhöhten baulichen Aufwand nach sich ziehen würde.

Die Schalteinrichtung 16 weist bei der erfindungsgemäßen Ausführungsform der Vorrichtung 10, wie sich insbesondere den Fig. 6 und 7 entnehmen lässt, des Weiteren eine Rückstelleinrichtung 52 auf, welche dem Betätigungselement 26 zugeordnet ist. Die Rückstelleinrichtung 52 beaufschlagt das Betätigungselement 26, derart, dass das Betätigungselement 26 selbsttätig aus der Kontaktstellung (nicht dargestellt) in die Ausgangsstellung entsprechend der Fig. 6 zurückverbringbar ist. Hierdurch ist sichergestellt, dass das Betätigungselement 26 ohne aktive Handhabung automatisch in die Ausgangsstellung zurückkehrt, somit der Motor zeitnah ausgeschaltet wird und die erfindungsgemäße Vorrichtung 10 insgesamt außer Betrieb gelangt.

Die Rückstelleinrichtung 52 weist in dem vorliegenden Ausführungsbeispiel der Vorrichtung 10 eine Magnetanordnung 54 auf. Die Magnetanordnung 54 umfasst einen Magnet 56 bestimmter Polarität, der an oder in dem Betätigungselement 26 angeordnet ist. Weiterhin ist die Magnetanordnung 54 mit zwei Magneten 58, 60 versehen, welche dem Magnet 56 zugewandt und in dem Gehäuse 12 angeordnet sind. Der Magnet 58 weist dabei eine Polarität auf, welche der Polarität des Magneten 56 entspricht. Der Magnet 60 hingegen weist eine Polarität auf, welche der Polarität jeweils der Magneten 56, 58 entgegengesetzt ist. Auf diese Weise versucht das Betätigungselement 26 entgegen der Haltekraft des Benutzers permanent aus der zweiten Stellung, d.h. Kontaktstellung, in die erste Stellung, d.h. Ausgangsstellung, zurückzugelangen.

Ohne im Einzelnen dargestellt zu sein, kann die Rückstelleinrichtung 52 selbstverständlich auch durch eine Federanordnung gebildet sein, wobei eine Feder (nicht gezeigt) zwischen dem Betätigungselement 26 der Schalteinrichtung 16 und dem Gehäuse 12 angeordnet ist. Durch die Feder, die eine Federkraft erzeugt, welche der Haltekraft des Benutzers entgegengesetzt ist, versucht das Betätigungselement 26 gleichsam in die Ausgangsstellung zurückzukehren.

Die Schalteinrichtung 16 ist darüber hinaus, wie aus den Fig. 1, 2 und 5 ersichtlich ist, mit einer Einrichtung 62 zum Anzeigen des aktuellen Zustandes der Stromversorgungseinrichtung 20, welche dem Motor 18 zugeordnet ist, versehen. Die Einrichtung 62 umfasst bei dem Ausführungsbeispiel nach den Fig. 1, 2 und 5 einen Gehäuseabschnitt 64, der lichtdurchlässig ist und im Inneren mit optischen Signalgebungselementen 66, insbesondere lichtemittierenden Dioden (LEDs), versehen ist (vgl. insbesondere Fig. 8A, 8B, 8D und 8E). Der lichtdurchlässige Gehäuseabschnitt 64 ist bei der Ausführung der Vorrichtung 10 nach den Fig. 1 bis 8E ringförmig, d.h. als Ring, ausgestaltet.

Beispielsweise ist es denkbar, dass weiße Signalgebungselemente 66 aktiviert sind, solange die Stromversorgungseinrichtung 20 einen Ladezustand zwischen 50 % und 100 % aufweist. Der ringförmige Gehäuseabschnitt 64 leuchtet somit weiß. Wenn der Ladezustand der Stromversorgungseinrichtung 20 einen Ladezustand zwischen 30 % und 50 % erreicht, werden zum Beispiel blaue Signalgebungselemente 66 aktiviert, wodurch der Gehäuseabschnitt 64 sodann blau leuchtet. Beträgt der Ladezustand der Stromversorgungseinrichtung 20 hingegen weniger als 30 %, werden automatisch rote Signalgebungselemente 66 angesteuert, welche den Gehäuseabschnitt 64 nur mehr rot leuchten lassen. Der Benutzer wird daher frühzeitig über den jeweils aktuellen Ladezustand der Stromversorgungseinrichtung 20 in Kenntnis gesetzt.

Des Weiteren ist das Gehäuse 12 bei der Ausführungsform der Vorrichtung 10 nach den Fig. 1 bis 8E gekapselt ausgebildet. Die Stromversorgungseinrichtung 20 weist dabei wenigstens einen wiederaufladbaren Akkumulator 68 auf. Der Akkumulator 68 ist über etwa punktförmige Kontakte 70, 72 an einem externen Ladegerät (nicht dargestellt) elektrisch anschließbar. Die Kontakte 70, 72 sind an der Außenseite 14, nämlich an der unteren Stirnseite 74, des Gehäuses 12 angeordnet. Um eine Wiederaufladung der Stromversorgungseinrichtung 20 in dem externen Ladegerät zu erreichen, sind gegebenenfalls zusätzliche konstruktive Maßnahmen, etwa Zentriereinrichtungen oder dergleichen, erforderlich, um eine entsprechende Kontaktierung zwischen Vorrichtung 10 und externen Ladegerät automatisch erreichen zu können.

Wie sich der schematischen Darstellung der Fig. 8A bis 8E entnehmen lässt, umfasst die Vorrichtung 10 nach der Erfindung darüber hinaus ein Relais 76, welches dem Motor 18 im Bereich der unteren Stirnseite 74 des Gehäuses 12 zugeordnet ist. Das Relais 76 ist zwischen einer Steuerplatine 78 und dem Magnetschalter 44, der seinerseits mit der Steuerplatine 78 elektrisch verbunden ist, angeordnet. Das Relais 76 sorgt einerseits für den Aufbau und die Aufrechterhaltung einer ausreichenden Betriebsspannung zum Motor 18, sobald der Magnetschalter 44 infolge einer Verdrehung des Betätigungselementes 26 aktiviert wird. Andererseits dient das Relais 76 in besonders vorteilhafter Weise als Bremse für den Motor 18. Insoweit ist ein Nachlaufen des Motors 18 nach Rückkehr des Betätigungselementes 26 in dessen Ausgangsstellung und damit nach Deaktivierung des Magnetschalters 44 sicher ausgeschlossen. Die Folge ist eine sehr exakte Handhabungsmöglichkeit der Vorrichtung 10 nach der Erfindung durch den Benutzer.

Zwischen der Stromversorgungseinrichtung 20 und der Steuerplatine 78 ist eine Isolierlage 80 vorgesehen, welche einer mechanischen und thermischen Trennung von Stromversorgungseinrichtung 20 und Steuerplatine 78 dient.

Wie vor allem aus den Fig. 8A, 8B und 8E ersichtlich ist, ist der Motor 18 an dessen Außenumfang mit einem Abschirmblech 82 oder dergleichen versehen, um eine elektromagnetische Abschirmung zwischen Motor 18 und Magnetschalter 44 zu erhalten.

Dem Motor 18 wiederum unmittelbar benachbart zugeordnet ist zusätzlich ein Getriebe 84, das zwischen Motor 18 und zapfenförmigem Antriebselement 22 zwischengeschaltet ist. Das Getriebe 84, das rein optional vorgesehen sein kann, sorgt für genau vordefinierte Drehzahlen und/oder Kraft- bzw. Momentenübertragungen, welche von dem Motor 18 auf das zapfenförmige Element 22 eingeleitet und von diesem wiederum auf die Instrumente oder Werkzeuge übertragen werden sollen. Das Antriebselement 22 kann beispielsweise mit einem im Querschnitt rechteckförmigen, quadratischen, sechseckförmigen oder sonstwie polygonalen, elliptischen, trapezoiden, parallelogrammartigen oder halbkreisförmigen Profil ausgestattet sein.

Über radial an dem Gehäuse 12 angeordnete Bohrungen 85 oder Ausnehmungen bzw. Vorsprünge (nicht gezeigt) und zu diesen korrespondierend ausgebildete Vorsprünge bzw. Bohrungen oder Ausnehmungen an feststehenden Bauteilen der Instrumente oder Werkzeuge (nicht dargestellt), die als Kupplung vorgesehen sind, sind die Vorrichtung 10 und die Instrumente oder Werkzeuge in gegenseitigem Wirkeingriff miteinander lösbar verbindbar.

Das zapfenförmige Antriebselement 22 selbst ist auf einem Kugellager 86 abgestützt und von diesem getragen. Das Kugellager 86 ist über einen Abstandsring 88 von einer Dichtung 90 auf Abstand gehalten. Die Dichtung 90 verschließt einen Ringspalt 92 an der oberen Stirnseite 24 des Gehäuses 12 und sorgt soweit für eine vollständige Abkapselung bzw. Verkapselung des Gehäuses 12 insgesamt.

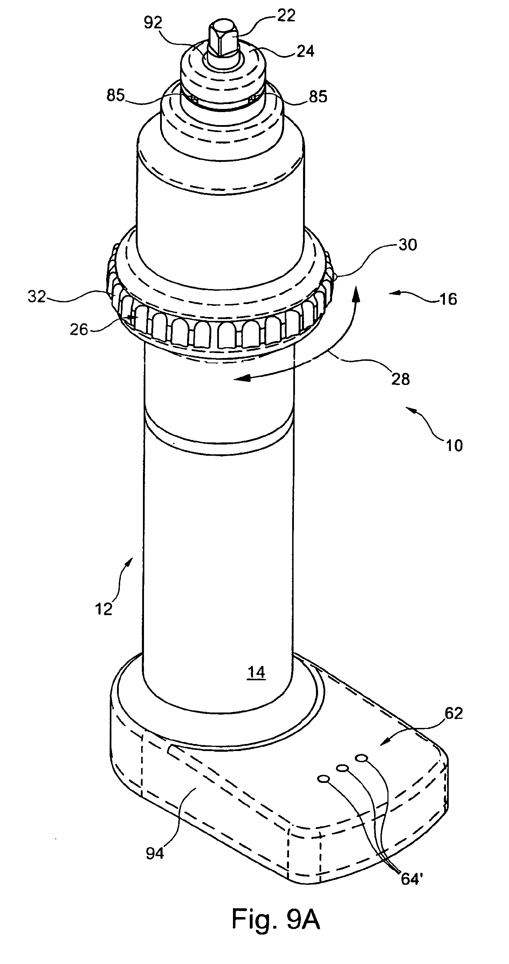

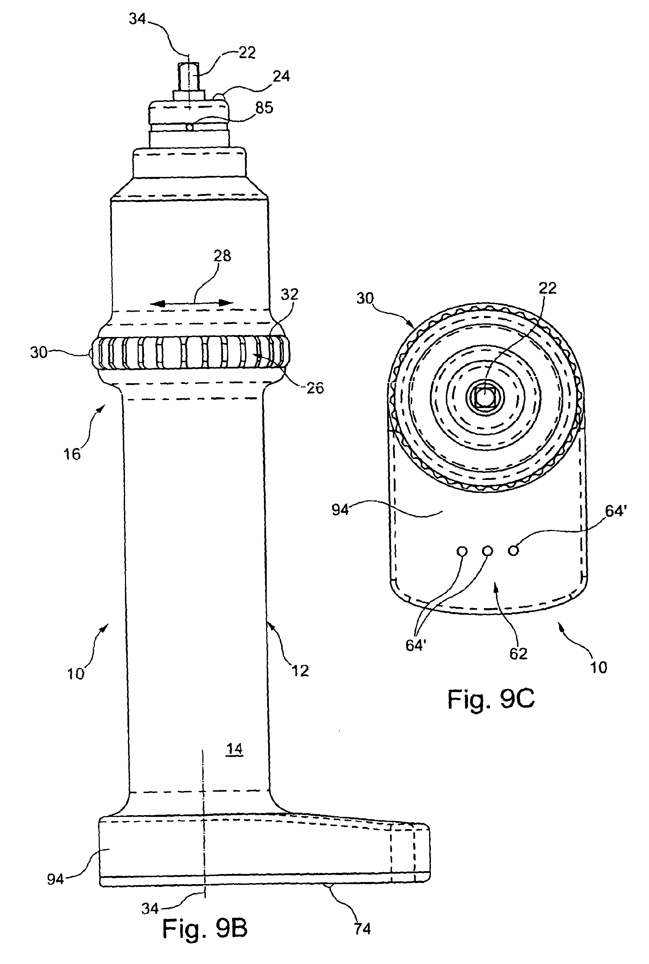

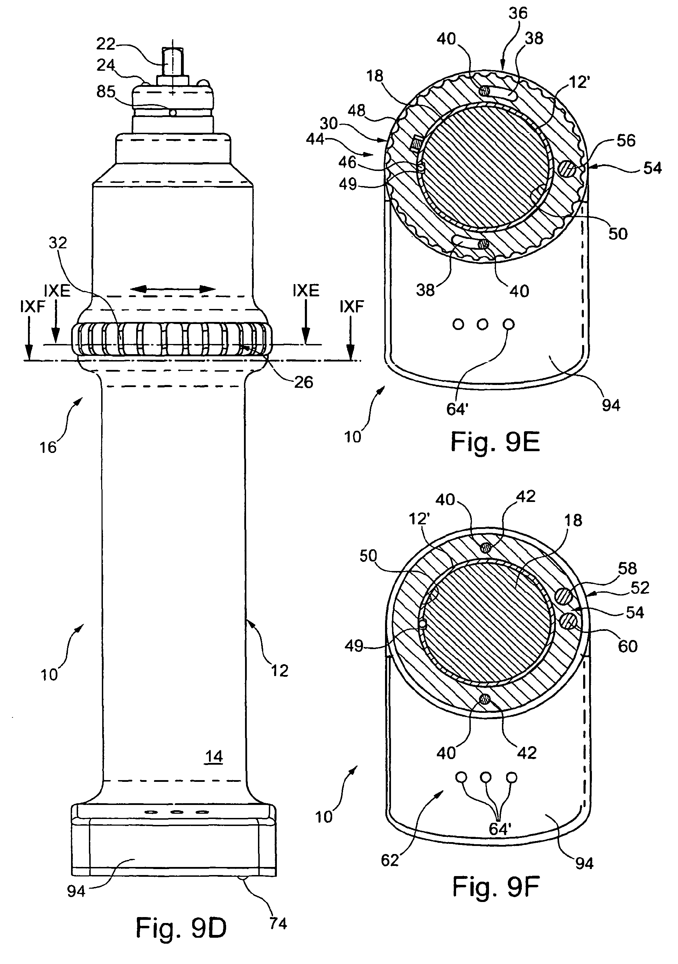

In den Fig. 9A bis 9F ist eine weitere Ausführungsform einer erfindungsgemäßen Vorrichtung 10 dargestellt. Die Ausführungsform nach den Fig. 9A bis 9F entspricht hinsichtlich ihrer Funktion im Wesentlichen der Ausführungsform der Vorrichtung 10 nach den Fig. 1 bis 8E.

Konstruktiv unterschiedlich sind allerdings die Abmessungen und Form der Vorrichtung 10 nach den Fig. 9A bis 9F.

So sind zum einen die Abmessungen der Ausführungsform der Vorrichtung 10 nach den Fig. 9A bis 9F proportional erheblich größer gewählt. Dies resultiert vornehmlich daraus, als die Vorrichtung 10 nach den Fig. 9A bis 9F mit einer Stromversorgungseinrichtung 20 ausgerüstet ist, die insgesamt drei wiederaufladbare Akkumulatoren 68 (nicht dargestellt) umfasst. Insoweit muss das Gehäuse 12 zwangsläufig größer ausgestaltet sein.

Zum anderen besitzt die Vorrichtung 10 der in den Fig. 9A bis 9F gezeigten Ausführungsform zusätzlich ein Fußelement 94 im Bereich der unteren Stirnseite 74 des Gehäuses 12. Das Fußelement 94 dient dabei einer automatischen Aufstellung bzw. Aufrichtung der Vorrichtung 10 während deren Benutzung. Zudem wirkt das Fußelement 94 einer möglichen selbsttätigen Bewegung der Vorrichtung 10 im Falle einer seitlichen Ablage der Länge nach entgegen.

Ein weiterer konstruktiver Unterschied zwischen der Ausführungsform der Vorrichtung 10 nach den Fig. 9A bis 9F gegenüber der Ausführungsform nach den Fig. 1 bis 8E bzw. 8A bis 8F besteht in der Ausgestaltung des lichtdurchlässigen Gehäuseabschnittes 64'. Der Gehäuseabschnitt 64' ist nämlich diskontinuierlich, insbesondere punktförmig, ausgebildet. So sind in dem Fußelement 94 insgesamt drei lichtdurchlässige, punktförmig ausgestaltete Gehäuseabschnitte 64' vorgesehen, welchen jeweils ein Signalgebungselement 66 unterschiedlicher Farbe zugeordnet ist. Die Farbgestaltung zur Anzeige des momentanen Ladezustandes der Stromversorgungseinrichtung 20 kann gleich derjenigen gewählt sein, wie bei den vorhergehenden Ausführungsformen. Ebenso ist es jedoch denkbar, eine andere Farbgestaltung, etwa diejenige einer Ampelschaltung, zu bevorzugen. So könnten die Farben Grün einen Ladezustand der Stromversorgungseinrichtung zwischen 50 % und 100 %, Gelb zwischen 30 % und 50 % und Rot weniger 30 % bis zu keiner vorhandenen Ladung mehr anzeigen.

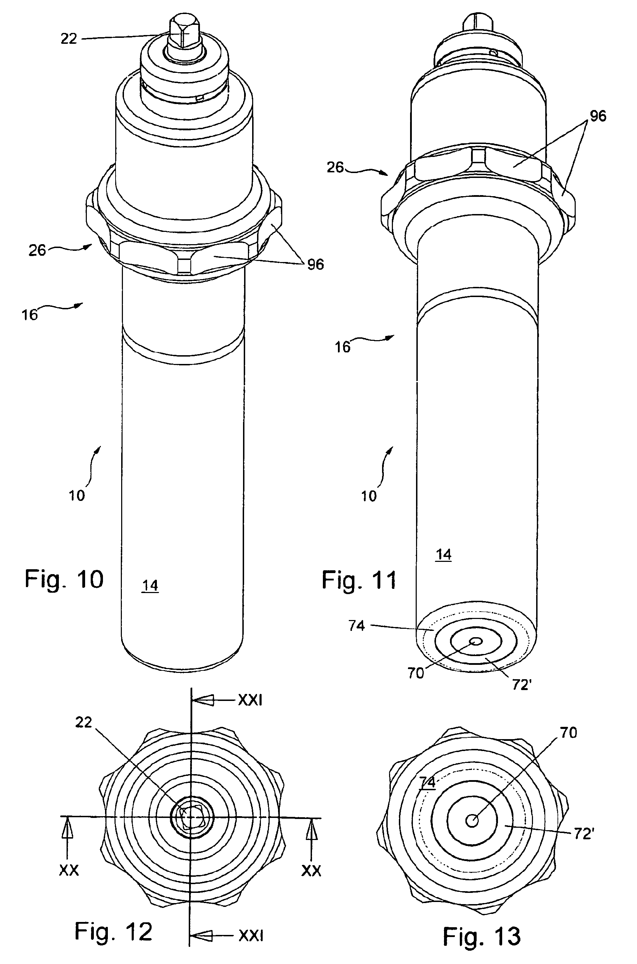

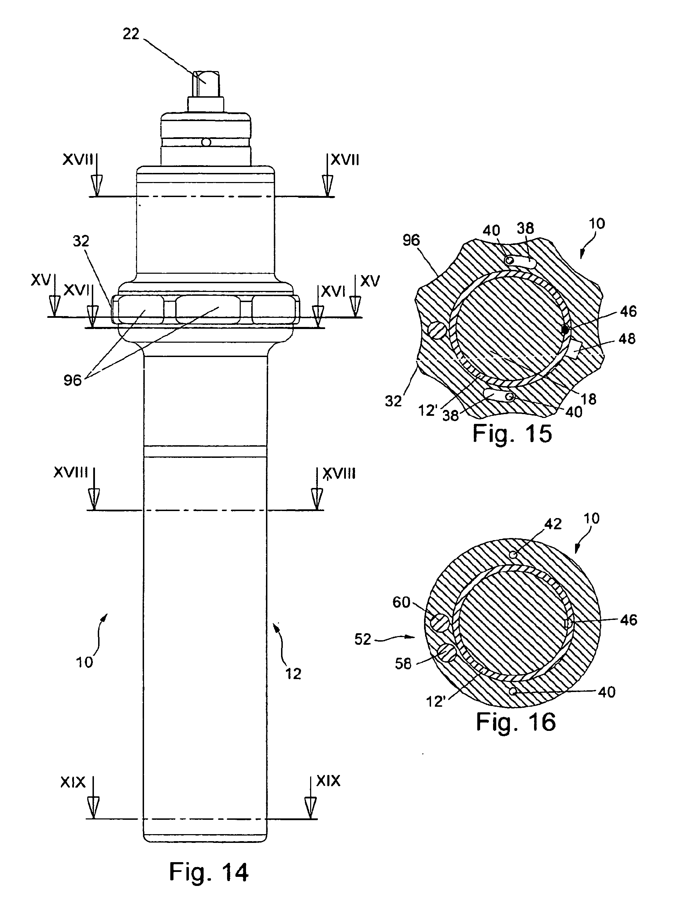

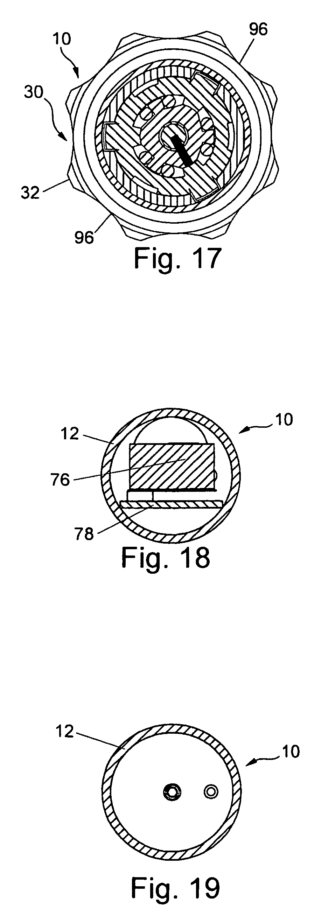

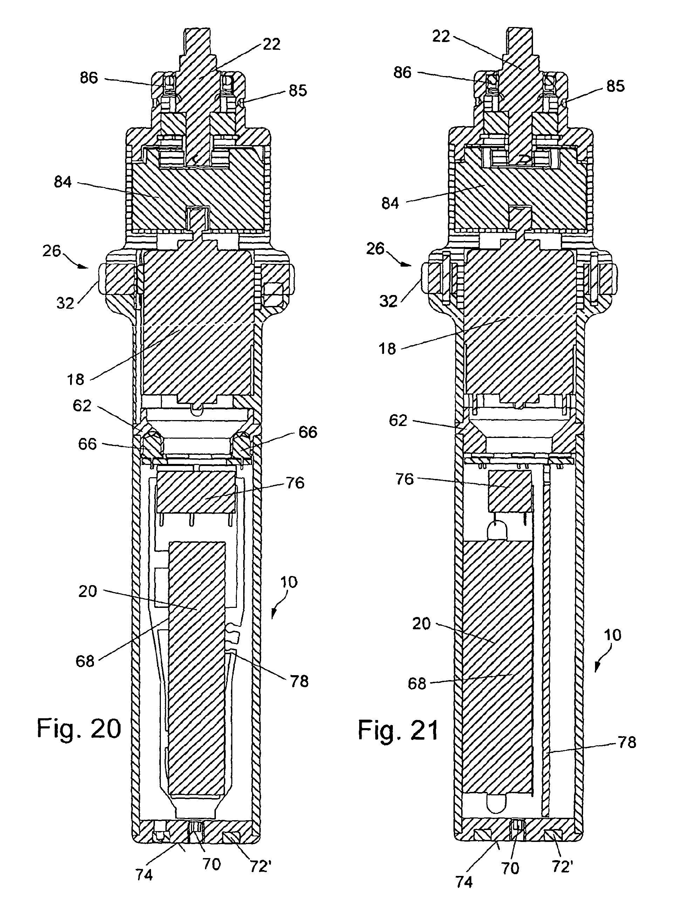

In den Fig. 10 bis 21 ist schließlich eine noch andere Ausführungsform einer erfindungsgemäßen Vorrichtung 10 dargestellt. Die Ausführungsform nach den Fig. 10 bis 21 entspricht hinsichtlich ihrer Funktion im Wesentlichen den Ausführungsformen der Vorrichtung 10 nach den Fig. 1 bis 8E bzw. 9A bis 9F.

Zudem gleicht die Ausführungsform der erfindungsgemäßen Vorrichtung 10 nach den Fig. 10 bis 21 in ihrer konstruktiven Ausgestaltung weitgehend derjenigen nach den Fig. 1 bis 8E, mit einigen, wenigen Ausnahmen.

Demnach ist das Betätigungselement 26 der Schalteinrichtung 16 an dessen Außenumfang 30 mit einer andersgearteten Profilierung 32 versehen. Die Profilierung 32 an dem Außenumfang 30 ist in Form von wenigstens zwei Griffmulden 96 oder dergleichen, welche an dem Außenumfang 30 des Betätigungselementes 26 voneinander gleich beabstandet angeordnet sind, ausgebildet. Bei einer solchen Ausgestaltung sind allerdings mehr als zwei Griffmulden 96 von Vorteil. Wie vor allem aus den Fig. 12 und 13 hervorgeht, sind bei der dargestellten Ausführungsform der Vorrichtung 10 nach den Fig. 12 bis 21 zweckmäßigerweise insgesamt 8 solcher Griffmulden 96 vorgesehen.

Des Weiteren kann bei der Ausführungsform der erfindungsgemäßen Vorrichtung 10 nach den Fig. 10 bis 21 eine Wiederaufladung des wenigstens einen Akkumulators 68 automatisch erfolgen, sobald die Vorrichtung 10 in eine entsprechende Ausnehmung des externen Ladegerätes eingesteckt wird. So ist der Kontakt 70, der beispielsweise als Plus-Pol ausgestaltet ist, mittig an der unteren Stirnseite 74 des Gehäuses 12 angeordnet. Anstelle eines punktförmigen Kontaktes 72 ist jedoch ein im Wesentlichen kreisringförmiger Kontakt 72' vorgesehen, der wiederum als Minus-Pol ausgeführt ist. Aufgrund der Anordnung und Ausbildung der Kontakte 70, 72' ist eine bestimmte Stellung bzw. Einführung der Vorrichtung 10 in der bzw. die Ausnehmung des externen Ladegerätes nicht erforderlich. Unterschiedlich zu der Ausführungsform der Vorrichtung 10 nach den Fig. 1 bis 8E ist schließlich noch die Anordnung des Relais 76 im Bereich der unteren Stirnseite 74 des Gehäuses 12. Bei der Ausführungsform der Vorrichtung 10 nach den Fig. 10 bis 21 ist das Relais 76 demgegenüber dem Motor 18 unmittelbar benachbart angeordnet, wodurch sich eine vereinfachte Bauweise insgesamt erhalten lässt.

Das Gehäuse 12 und/oder die Schalteinrichtung 16 und/oder das Betätigungselement 26 und/oder der lichtdurchlässige Gehäuseabschnitt 64, 69' ist/sind vorzugsweise aus Kunststoff, insbesondere (Hart-)Polyvinylchlorid Polyoximethylen (POM), Polyester, Polyphenylensulfon (PPSU), ABS, Acryl, Polycarbonat, Tetrafluorethylen oder Impax, duroplastischen Elastomeren, mit oder ohne Glasfaserverstärkung, oder einer Kombination hieraus gebildet.

Alternativ oder kumulativ kann/können das Gehäuse 12 und/oder die Schalteinrichtung 16 und/oder das Betätigungselement 26 auch aus Metall, insbesondere (nicht-rostendem) Stahl, Edelstahl, Aluminium, Messing, Zink, Rotgusslegierungen oder einer Legierung hieraus gebildet sein.

Das Gehäuse 12 ist schließlich mittels Fräsens, Spritzgießens oder Laser herstellbar.

Die Erfindung ist nicht auf die vorhergehenden Ausführungsformen beschränkt. Vielmehr ist es ebenso möglich, anstelle eines gekapselten Gehäuses 12 und einer Stromversorgungseinrichtung 20 mit wenigstens einem wiederaufladbaren Akkumulator 64 ein Gehäuse mit einem Schraub-, Bajonette- oder dergleichen -verschluss mit einem deckel- oder kappenförmigen Verschlusselement abgedichtet verschließbar auszugestalten, wobei die Stromversorgungseinrichtung 20 dann wenigstens eine Batterie aufweist. Auf diese Weise lässt sich ebenso eine vielfach wieder verwendbare Vorrichtung 10 erreichen. Alternativ dazu ist es ebenso denkbar, das Gehäuse 12 gekapselt auszubilden, wobei die Stromversorgungseinrichtung 20 jedoch eine Leitung zur elektrischen Verbindung mit einer externen Stromquelle aufweist (ebenfalls nicht dargestellt).

## Patentansprüche

1. Vorrichtung zum Antrieb von chirurgischen Instrumenten, die ein Gehäuse (12) zur Aufnahme eines Motors (18), den Motor (18), eine dem Motor (18) zugeordnete Stromversorgungseinrichtung (20) und ein dem Motor (18) zugeordnetes, teilweise aus dem Gehäuse (12) herausragendes und mit den Instrumenten zusammenwirkendes, zapfenförmiges Antriebselement (22) sowie eine an der Außenseite (14) des Gehäuses (12) angeordnete Schalteinrichtung (16) zum Einschalten und Ausschalten des Motors (18) aufweist, wobei die Schalteinrichtung (16) ein Betätigungselement (26) aufweist, das sich vollständig um den Umfang des Gehäuses (12) erstreckt und gegenüber dem Gehäuse (12) relativ verdrehbar ausgebildet ist, **dadurch gekennzeichnet, dass** die Schalteinrichtung (16) einen durch das Betätigungselement (26) betätigbaren Magnetschalter (44) zum Einschalten und Ausschalten des Motors (18) und ein dem Motor (18) zugeordnetes und als Motorbremse sowie zum Aufbau und zur Aufrechterhaltung einer ausreichenden Betriebsspannung vorgesehenes Relais (76) umfasst.

2. Vorrichtung nach Anspruch 1, **dadurch gekennzeichnet, dass** das Betätigungselement (26) der Schalteinrichtung (16) ring- oder scheibenförmig, insbesondere als Ring ausgebildet ist, der vorzugsweise an einem Teil (12') des Gehäuses (12) abgestützt ist, ausgebildet ist.

3. Vorrichtung nach Anspruch 1 oder 2, **dadurch gekennzeichnet, dass** sich das Betätigungselement (26) der Schalteinrichtung (16) über die Außenseite (14) des Gehäuses (12) hinauserstreckt.

4. Vorrichtung nach Anspruch 3, **dadurch gekennzeichnet, dass** das Betätigungselement (26) der Schalteinrichtung (16) an dessen Außenumfang (30) mit einer Profilierung (32) versehen ist, die insbesondere als Rändelung, Zahnrad, Zahnkranz oder in Form von wenigstens zwei Griffmulden (96) oder dergleichen ausgebildet ist.

5. Vorrichtung nach einem der Ansprüche 1 bis 4, **dadurch gekennzeichnet, dass** das Betätigungselement (26) der Schalteinrichtung (16) gegenüber dem Gehäuse (12) um die Längsachse (34) des Gehäuses (12) oder eine dazu parallele Achse relativ verdrehbar ausgebildet ist.

6. Vorrichtung nach einem der Ansprüche 1 bis 5, **dadurch gekennzeichnet, dass** das Betätigungselement (26) der Schalteinrichtung (16) aus einer ersten Stellung, insbesondere Ausgangsstellung, in eine zweite Stellung, insbesondere Kontaktstellung, und umgekehrt, gegenüber dem Gehäuse (12) relativ verdrehbar ist.

7. Vorrichtung nach einem der Ansprüche 1 bis 6, **dadurch gekennzeichnet, dass** dem Betätigungselement (26) der Schalteinrichtung (16) wenigstens ein Anschlagelement (36) zur Begrenzung der relativen Verdrehbarkeit des Betätigungselementes (26) gegenüber dem Gehäuse (12) zugeordnet ist.

8. Vorrichtung nach einem der Ansprüche 1 bis 7, **dadurch gekennzeichnet, dass** das Betätigungselement (26) der Schalteinrichtung (16) mit wenigstens einem/einer im Wesentlichen bogenförmigen Langloch (38) oder Ausnehmung versehen ist, in dem/der ein sich im Wesentlichen parallel zu der Längsachse (34) des Gehäuses (12) verlaufender Stift (40), Bolzen oder dergleichen Führungselement aufgenommen ist.

9. Vorrichtung nach Anspruch 8, **dadurch gekennzeichnet, dass** das Betätigungselement (26) der Schalteinrichtung (16) mit zwei im Wesentlichen bogenförmigen Langlöchern (38) oder Ausnehmungen versehen ist, die insbesondere im Wesentlichen um etwa 180° zueinander versetzt angeordnet sind.

10. Vorrichtung nach einem der Ansprüche 1 bis 9, **dadurch gekennzeichnet, dass** die Schalteinrichtung (16) einen Reed-Schalter zum Einschalten und Ausschalten des Motors (18) umfasst.

11. Vorrichtung nach einem der Ansprüche 1 bis 10, **dadurch gekennzeichnet, dass** die Schalteinrichtung (16) einen Kontaktdraht (46) zur elektrischen Verbindung des Motors (18) mit der Stromversorgungseinrichtung (20) und einen mit dem Kontaktdraht (46) zusammenwirkenden Magnet (48) umfasst, wobei insbesondere der Kontaktdraht (46) der Schalteinrichtung (16) im Wesentlichen in einer sich etwa parallel zur Längsachse (34) des Gehäuses (12) erstreckenden Nut (49) und der Magnet (48) an dem Betätigungselement (26) der Schalteinrichtung (16) an dessen Innenumfang (50) dem Kontaktdraht (46) zugewandt angeordnet sind, und umgekehrt.

12. Vorrichtung nach einem der Ansprüche 1 bis 11, **dadurch gekennzeichnet, dass** die Schalteinrichtung (16) eine Rückstelleinrichtung (52) umfasst, welche dem Betätigungselement (26) zugeordnet ist und das Betätigungselement (26) beaufschlagt, derart, dass das Betätigungselement (26) selbsttätig aus der Kontaktstellung in die Ausgangsstellung zurück verbringbar ist.

13. Vorrichtung nach Anspruch 12, **dadurch gekennzeichnet, dass** die Rückstelleinrichtung (52) eine Magnetanordnung (54) aufweist, wobei ein Magnet (56) an oder in dem Betätigungselement (26) der Schalteinrichtung (16) angeordnet ist und zwei dem einen Magnet (56) zugewandte Magnete (58, 60) in dem Gehäuse (12) angeordnet sind, oder dass die Rückstelleinrichtung (52) eine Federanordnung aufweist, wobei eine Feder zwischen dem Betätigungselement (26) der Schalteinrichtung (16) und dem Gehäuse (12) angeordnet ist.

14. Vorrichtung nach einem der Ansprüche 1 bis 13, **dadurch gekennzeichnet, dass** die Schalteinrichtung (16) eine Einrichtung (62) zum Anzeigen des aktuellen Zustandes der dem Motor (18) zugeordneten Stromversorgungseinrichtung (20) umfasst.

15. Vorrichtung nach einem der Ansprüche 1 bis 14, **dadurch gekennzeichnet, dass** die Schalteinrichtung (16) wenigstens einen lichtdurchlässigen Gehäuseabschnitt (64, 64'), der mit optischen Signalgebungselementen (66), insbesondere lichtemittierenden Dioden, versehen ist, umfasst, wobei insbesondere der wenigstens eine lichtdurchlässige Gehäuseabschnitt (64) kontinuierlich, insbesondere ringförmig, und/oder (64') diskontinuierlich, insbesondere punktförmig, ausgebildet ist.

16. Vorrichtung nach einem der Ansprüche 1 bis 15, **dadurch gekennzeichnet, dass** das Gehäuse (12) gekapselt ausgebildet ist, wobei die Stromversorgungseinrichtung (20) wenigstens einen wiederaufladbaren Akkumulator (68) aufweist.

17. Vorrichtung nach Anspruch 16, **dadurch gekennzeichnet, dass** der wenigstens eine wiederaufladbare Akkumulator (68) über an einer Außenseite (14) des Gehäuses (12) angeordnete Kontakte (70, 72, 72'), insbesondere einen kreisringförmigen Kontakt (72'), an einem externen Ladegerät elektrisch anschließbar ist.

18. Vorrichtung nach einem der Ansprüche 1 bis 17, **dadurch gekennzeichnet, dass** das Gehäuse (12) gekapselt ausgebildet ist, wobei die Stromversorgungseinrichtung (20) eine Leitung zur elektrischen Verbindung mit einer externen Stromquelle aufweist, oder dass das Gehäuse (12) über einen Schraub-, Bayonette- oder dergleichen -verschluss mit einem deckel- oder kappenförmigen Verschlusselement abgedichtet verschließbar ist, wobei die Stromversorgungseinrichtung (20) wenigstens eine Batterie aufweist.

19. Vorrichtung nach einem der Ansprüche 1 bis 18, **dadurch gekennzeichnet, dass** das Gehäuse (12) und/oder die Schalteinrichtung (16) und/oder das Betätigungselement (26) und/oder der lichtdurchlässige Gehäuseabschnitt (64, 64') aus Kunststoff, insbesondere (Hart-)Polyvinylchlorid Polyoximethylen (POM), Polyester, Polyphenylensulfon (PPSU), ABS, Acryl, Polycarbonat, Tetrafluorethylen oder Impax, duroplastischen Elastomeren, mit oder ohne Glasfaserverstärkung, oder einer Kombination hieraus gebildet ist/sind, und/oder dass das Gehäuse (12) und/oder die Schalteinrichtung (16) und/oder das Betätigungselement (26) aus Metall, insbesondere Stahl, insbesondere nicht-rostendem, Edelstahl, Aluminium, Messing, Zink, Rotgusslegierungen oder einer Legierung hieraus gebildet ist/sind.

20. Vorrichtung nach einem der Ansprüche 1 bis 19, **dadurch gekennzeichnet, dass** das Gehäuse (12) mittels Fräsens, Spritzgießens oder Laser herstellbar ist.

## Claims

1. Device for driving surgical instruments, having a housing (12) for mounting a motor (18), the motor (18), a power supply device (20) associated with the motor (18) and a peg-shaped drive element (22) partially protruding out of the housing (12), associated with the motor (18) and interacting with the instruments, and a switching device (16) located on the outside (14) of the housing (12) for switching the motor (18) on and off, while the switching device (16) comprises an actuating element (26) that is fully extending around the circumference of the housing (12) and is designed in such a way that it is rotatable relative to the housing (12), **characterized in that** the switching device (16) comprises a magnetic switch (44) to be actuated by the actuating element (26) for switching the motor (18) on and off, and a relay (76) associated with the motor (18) and acting as a motor brake and for building up and maintaining a sufficient operating voltage to the motor (18).

2. Device in accordance with claim 1, **characterized in that** the actuating element (26) of the switching device (16) is designed in a ring or disc-shaped fashion, in particular is designed as a ring, which is in particular supported by one part (12') of the housing (12).

3. Device in accordance with claim 1 or 2, **characterized in that** the actuating element (26) of the switching device (16) is protruding beyond the outside (14) of the housing (12).

4. Device in accordance with claim 3, **characterized in that** the actuating element (26) of the switching device (16) is provided with a profile (32) on its outer circumference (30), in particular is designed as a knurling, gear-wheel or sprocket, or in form of at least two recessed grips (96) or similar.

5. Device in accordance with any of the claims 1 to 4, **characterized in that** the actuating element (26) of the switching device (16) is designed in such a way that it is rotatable relative to the housing (12) around the longitudinal axis (34) of the housing (12) or any axis parallel to it.

6. Device in accordance with any of the claims 1 to 5, **characterized in that** the actuating element (26) of the switching device (16) is rotatable relative to the housing (12) from a first position, in particular home position, into a second position, in particular contact position, and vice-versa.

7. Device in accordance with any of the claims 1 to 6, **characterized in that** the actuating element (26) of the switching device (16) is associated with at least one stop element (36) for limiting the relative rotatability of the actuating element (26) in relation to the housing (12).

8. Device in accordance with any of the claims 1 to 7, **characterized in that** the actuating element (26) of the switching device (16) is provided with at least one essentially arc-shaped oblong hole (38) or recess which houses a pin (40), bolt or similar guidance element running essentially parallel to the longitudinal axis (34) of the housing (12).

9. Device in accordance with claim 8, **characterized in that** the actuating element (26) of the switching device (16) is provided with two essentially arc-shaped oblong holes (38) or recesses, which in particular are essentially arranged at 180 degrees to each other.

10. Device in accordance with any of the claims 1 to 9, **characterized in that** the switching device (16) comprises a reed switch for switching the motor (18) on and off.

11. Device in accordance with any of the claims 1 to 10, **characterized in that** the switching device (16) comprises a contact wire (46) as an electrical connection of the motor (18) with the power supply device (20), and a magnet (48) interacting with the contact wire (46), with in particular the contact wire (46) of the switching device (16) is located essentially within a groove (49) running roughly parallel to the longitudinal axis (34) of the housing (12), and the magnet (48) on the actuating element (26) of the switching device (16) is located at its inner circumference (50) facing the contact wire (46), and vice-versa.

12. Device in accordance with any of the claims 1 to 11, **characterized in that** the switching device (16) comprises a resetting device (52) which is associated with the actuating element (26) and acts on the actuating element (26) in such a way that the actuating element (26) can move back automatically from the contact position into the home position.

13. Device in accordance with claim 12, **characterized in that** the resetting device (52) has a magnet arrangement (54), wherein one magnet (56) is arranged on or inside the actuating element (26) of the switching device (16), and two magnets (58, 60) facing the one magnet (56) are arranged inside the housing (12), or **in that** the resetting device (52) features a spring setup, wherein one spring is arranged between the actuating element (26) of the switching device (16) and the housing (12).

14. Device in accordance with any of the claims 1 to 13, **characterized in that** the switching device (16) comprises a device (62) for displaying the current status of the power supply device (20) associated with the motor (18).

15. Device in accordance with any of the claims 1 to 14, **characterized in that** the switching device (16) comprises at least one transparent housing section (64, 64') that is fitted with optical signal elements (66), in particular light-emitting diodes, with in particular the at least one transparent housing section is designed (64) continuously, in particular ring-shaped, and/or (64') discontinuously, in particular point-shaped.

16. Device in accordance with any of the claims 1 to 15, **characterized in that** the housing (12) is designed in a sealed fashion, wherein the power supply device (20) features at least one rechargeable battery (68).

17. Device in accordance with claim 16, **characterized in that** the at least one rechargeable battery (68) can be connected electrically via contacts (70, 72, 72'), in particular a circular contact (72'), arranged on an outside (14) of the housing (12) to an external charging device.

18. Device in accordance with any of the claims 1 to 17, **characterized in that** the housing (12) is designed in a sealed fashion, wherein the power supply device (20) features a cable for the electrical connection to an external power source, or **in that** the housing (12) can be sealed via a screw, bayonet or similar locking device with a cap or lid-shaped closing element, while the power supply device (20) has at least one battery.

19. Device in accordance with any of the claims 1 to 18, **characterized in that** the housing (12) and/or the switching device (16) and/or the actuating element (26) and/or the transparent housing section (64, 64') is/are made of plastic, in particular (hard) polyvinylchloride, polyoximethylene (POM), polyester, polyphenylensulfone (PPSU), ABS, acrylic, polycarbonate, tetrafluorethylene or impax, duroplastic elastomers, with or without fibreglass reinforcement, or any combination of the above, and/or **in that** the housing (12) and/or the switching device (16) and/or the actuating element (26) is/are made of metal, in particular steel, in particular (stainless) high-grade steel, aluminium, brass, zinc, red bronze alloys or any alloy of the above.

20. Device in accordance with any of the claims 1 to 19, **characterized in that** the housing (12) can be manufactured by means of machining, injection moulding or laser.

## Revendications

1. Dispositif pour l'entraînement d'instruments chirurgicaux, qui comprend un carter (12) destiné à recevoir un moteur (18), le moteur (18), un système d'alimentation électrique (20) associé au moteur (18), et un élément d'entraînement (22) en forme de tenon en saillie hors du carter (12) et coopérant avec les instruments, ainsi qu'un dispositif de commutation agencé sur la face extérieure (14) du carter (12) pour mettre en marche le moteur (18) et pour l'arrêter, dans lequel le dispositif de commutation (16) comprend un élément d'actionnement (26), qui s'étend entièrement sur la périphérie du carter (12) et est réalisé avec possibilité de rotation par rapport au carter (12), **caractérisé en ce que** le dispositif de commutation (16) comprend un commutateur magnétique (44), susceptible d'être actionné par l'élément d'actionnement (26), pour mettre en marche le moteur (18) et l'arrêter, et un relais (76) associé au moteur (18) est prévu à titre de frein moteur ainsi que pour l'établissement et le maintien d'une tension de service suffisante.

2. Dispositif selon la revendication 1, **caractérisé en ce que** l'élément d'actionnement (26) du dispositif de commutation (16) est réalisé en forme de bague ou en forme de disque, en particulier sous la forme d'une bague qui est de préférence soutenue sur une partie (12') du carter (12).

3. Dispositif selon la revendication 1 ou 2, **caractérisé en ce que** l'élément d'actionnement (26) du dispositif de commutation (16) s'étend au-delà de la face extérieure (14) du carter (12).

4. Dispositif selon la revendication 3, **caractérisé en ce que** l'élément d'actionnement (26) du dispositif de commutation (16) est doté à sa périphérie extérieure (30) d'un profilage (32), lequel est réalisé en particulier sous forme de moletage, de roue dentée, de couronne dentée, ou encore sous la forme d'au moins deux creux de préhension (96) ou similaire.

5. Dispositif selon l'une des revendications 1 à 4, **caractérisé en ce que** l'élément d'actionnement (26) du dispositif de commutation (16) est pivotant par rapport au carter (12) autour de l'axe longitudinal (34) du carter (12) ou d'un axe parallèle à celui-ci.

6. Dispositif selon l'une des revendications 1 à 5, **caractérisé en ce que** l'élément d'actionnement (26) du dispositif de commutation (16) peut être tourné par rapport au carter (12) depuis une première position, en particulier une position de départ, vers une seconde position, en particulierune position de contact, et inversement.

7. Dispositif selon l'une des revendications 1 à 6, **caractérisé en ce qu'**au moins un élément de butée (36), destiné à limiter la capacité de rotation relative de l'élément d'actionnement (26) par rapport au carter (12), est associé à l'élément d'actionnement (26).

8. Dispositif selon l'une des revendications 1 à 7, **caractérisé en ce que** l'élément d'actionnement (26) du dispositif de commutation (16) est pourvu d'au moins un trou oblong (38) ou d'une échancrure sensiblement en forme d'arc, dans lequel/laquelle est reçue une tige (40), un goujon, ou un élément de guidage analogue, qui s'étend sensiblement parallèlement à l'axe longitudinal (34) du carter (12).

9. Dispositif selon la revendication 8, **caractérisé en ce que** l'élément d'actionnement (26) du dispositif de commutation (16) est pourvu de deux trous oblongs (38) ou échancrures sensiblement en forme d'arc, qui sont agencé(e)s en particulier sensiblement en décalage d'environ 180° l'un/l'une par rapport à l'autre.

10. Dispositif selon l'une des revendications 1 à 9, **caractérisé en ce que** le dispositif de commutation (16) comprend un commutateur de type "Reed" pour mettre en marche le moteur (18) et pour l'arrêter.

11. Dispositif selon l'une des revendications 1 à 10, **caractérisé en ce que** le dispositif de commutation (16) comprend un fil de contact (46) pour la connexion électrique du moteur (18) avec le système d'alimentation électrique (20) et un élément (48) qui coopère avec le fil de contact (46), dans lequel en particulier le fil de contact (46) du dispositif de commutation (16) est agencé sensiblement dans une rainure (49) qui s'étend approximativement parallèlement à l'axe longitudinal (34) du carter (12), et l'élément (48) est agencé sur l'élément d'actionnement (26) du dispositif de commutation (16) sur sa périphérie intérieure (50) en étant tourné vers le fil de contact (46), et inversement.

12. Dispositif selon l'une des revendications 1 à 11, **caractérisé en ce que** le dispositif de commutation (16) comprend un moyen de rappel (52), qui est associé à l'élément d'actionnement (26) et qui sollicite l'élément d'actionnement (26) de telle façon que l'élément d'actionnement (26) peut être amené automatiquement hors de la position de contact en retour dans la position de départ.

13. Dispositif selon la revendication 12, **caractérisé en ce que** le moyen de rappel (52) comprend un agencement à aimant (54), de telle sorte qu'un élément (56) est agencé sur ou dans l'élément d'actionnement (26) du dispositif de commutation (16), et deux aimants (58, 60), tournés vers l'aimant (56), sont agencés dans le carter (12), ou **en ce que** le moyen de rappel (52) comprend un agencement à ressort, dans lequel un ressort et agencé entre l'élément d'actionnement (26) du dispositif de commutation (16) et le carter (12).

14. Dispositif selon l'une des revendications 1 à 13, **caractérisé en ce que** le dispositif de commutation (16) comprend un moyen (62) pour indiquer la situation actuelle du système d'alimentation électrique (20) associé au moteur (18).

15. Dispositif selon l'une des revendications 1 à 14, **caractérisé en ce que** le dispositif de commutation (16) comprend au moins un tronçon de carter (64, 64') transparent, qui est pourvu d'éléments de signalisation optique (66), en particulier de diodes électroluminescentes, et ledit au moins un tronçon de carter transparent (64) est réalisé en particulier de façon continue, en particulier en forme de bague et/ou (64') est réalisé de façon discontinue, en particulier sous forme ponctuelle.

16. Dispositif selon l'une des revendications 1 à 15, **caractérisé en ce que** le carter (16) est réalisé de manière encapsulée, et le système d'alimentation électrique (20) comprend au moins un accumulateur rechargeable (68).

17. Dispositif selon la revendication 16, **caractérisé en ce que** ledit au moins un accumulateur rechargeable (68) est susceptible d'être branché électriquement à un appareil de charge externe, via des contacts (70, 72, 72'), en particulier un contact (72') de forme circulaire, agencé sur une face extérieure (14) du carter (12).

18. Dispositif selon l'une des revendications 1 à 17, **caractérisé en ce que** le carter (12) est réalisé de manière encapsulée, et le système d'alimentation électrique (20) comprend une ligne pour la connexion électrique avec une source de courant extérieure, ou **en ce que** le carter (12) est susceptible d'être obturé de manière étanche avec un élément de fermeture en forme de couvercle ou en forme de capot via une fermeture à vis, à baïonnette ou similaire, ledit système d'alimentation électrique (20) comprenant au moins une pile/batterie.

19. Dispositif selon l'une des revendications 1 à 18, **caractérisé en ce que** le carter (12) et/ou le dispositif de commutation (16) et/ou l'élément d'actionnement (26) et/ou le tronçon de carter transparent (64, 64') est/sont réalisé(s) en matière plastique, en particulier en chlorure de polyvinyle (dur)-polyoxyméthylène (POM), polyester, polyphénylène sulfone (PPSU), ABS, acryl, polycarbonate, tétrafluoroéthylène ou Impax, élastomère thermodurcissable, avec ou sans renforcement en fibres de verre, ou une combinaison de ces matériaux, et/ou **en ce que** le carter (12) et/ou le dispositif de commutation (16) et/ou l'élément d'actionnement (26) est/sont réalisé(s) en métal, en particulier en acier, en particulier en acier inoxydable, en acier spécial, aluminium, laiton, zinc, alliage de fonte rouge, ou un alliage de ces matériaux.

20. Dispositif selon l'une des revendications 1 à 19, **caractérisé en ce que** le carter (12) est susceptible d'être fabriqué par fraisage, par moulage par injection ou par usinage au laser.
